# EUROPEAN PATENT SPECIFICATION

(11) **EP 4 673 052 B1**
(45) Date of publication and mention of the grant of the patent: **29.04.2026**
(21) Application number: 24775916.0
(22) Date of filing: 24.09.2024
(51) Int. Cl.: A61B 6/02, A61B 6/40, A61B 6/42, A61B 6/00

(54) **SPECTRALLY GENERATED STEREO X-RAY IMAGING**
SPEKTRAL ERZEUGTE STEREO-RÖNTGENBILDGEBUNG
IMAGERIE PAR RAYONS X STÉRÉO GÉNÉRÉE SPECTRALEMENT

(30) Priority: 28.09.2023 EP 23200288
(43) Date of publication of application: 07.01.2026
(73) Proprietor: Koninklijke Philips N.V., 5656 AG Eindhoven (NL)
(72) Inventor: KROENKE-HILLE, Sven, 5656 AG Eindhoven (NL); YOUNG, Stewart Matthew, 5656 AG Eindhoven (NL)
(74) Representative: Philips Intellectual Property & Standards
(86) International application number: PCT/EP2024/076708
(87) International publication number: WO 2025/068135

(56) References cited:
- JP-A- S63 246 738
- US-A1- 2019 105 003

## Description

### FIELD OF THE INVENTION

The present invention relates to X-ray imaging, and in particular to an X-ray device, to an X-ray system, and to an X-ray imaging method.

### BACKGROUND OF THE INVENTION

X-ray imaging is often the entry point to the diagnostic pathway for a patient. Follow-up three-dimensional (3D) exams like Computed Tomography (CT) and Magnetic Resonance (MR) may be frequently ordered to resolve ambiguities in the interpretation of findings in a two-dimensional (2D) X-ray image. Such exams may be expensive and come with additional dose for the patients in the case of CT.

Spectral CT systems are currently being developed whereby the resolution of different energies of X-ray photons is opening up a wide range of novel, previously unforeseen diagnostic imaging applications. This ability to resolve different wavelengths may enable a deeper anatomical and physiological understanding, since fundamental physical properties of tissue processes and osseous anatomy in the human body at different parts of the spectrum can be revealed thereby. A relatively simple example of the use of such techniques in planar radiography is the application of dual energy imaging for bone suppression in chest X-ray.
US2019105003 discloses an X-ray device, comprising: an X-ray generator ; and- an X-ray detector ; wherein the X-ray generator is configured to generate a polychromatic X-ray beam in a frequency range for an subject to be imaged ; and wherein the X-ray detector is configured to acquire a plurality of energy-resolved absorption images of the subject, which are usable for generating a stereo-absorption image of the subject.

### SUMMARY OF THE INVENTION

There may be a need to provide an improved X-ray device.

The object of the present invention is solved by the subject-matter of the independent claims, wherein further embodiments are incorporated in the dependent claims. It should be noted that the following described aspects of the invention apply also for the X-ray device, the X-ray system, and the X-ray imaging method.

According to a first aspect of the present invention, there is provided an X-ray device. The X-ray device comprises an X-ray generator and an X-ray detector. The X-ray generator is configured to generate a polychromatic X-ray beam in a frequency range for a subject to be imaged. The polychromatic X-ray beam is configured to have an energy dependent angulation with respect to the X-ray detector. The X-ray detector is configured to acquire a plurality of energy-resolved absorption images of the subject, which are usable for generating a stereo-absorption image of the subject.

Accordingly, a novel X-ray device is proposed, which may fill the gap between the purely 2D X-ray and the 3D CT or 3D MR modality while being far less technically complex as a CT or MR scanner and easier to use as compared to conventional X-ray tomosynthesis. The proposed X-ray device utilizes an energy dependent angulation of X-ray rays with respect to a spectral detector for generating a stereo-absorption image of an object. Different realizations of such an energy dependent X-ray angulations will be described in detail hereinafter. All implementations have in common that they are based on a single X-ray source and a single detector where no component is set into motion as compared to e.g., C-arm systems or CT scanners. Thereby, the setup is relatively simple and can be realized much cheaper as compared to e.g. a C-arm system. Moreover, its usage may integrate seamlessly into the conventional X-ray workflow.

As will be described hereinafter, some of the proposed realizations allow for varying the central beam relative to the spectral detector (i.e. the viewing direction) after the image acquisition. Thereby, the radiologist may retrospectively tune the viewing direction within a certain range in order to resolve ambiguities being present in a single 2D projective image or in order to correct for suboptimal patient positioning. Moreover, the proposed technology may potentially be used to speed-up and/or enhance CT or C-arm systems.

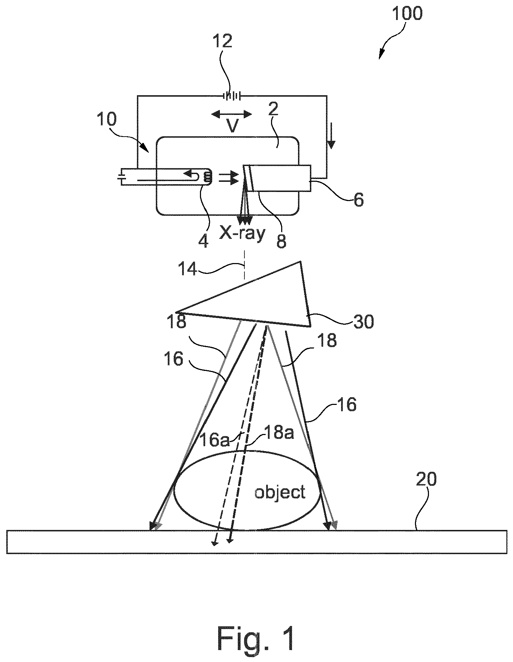

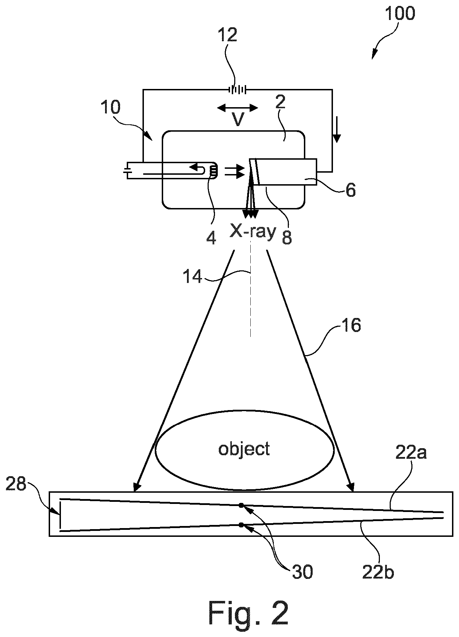

Accordingly, the proposed X-ray device, such as the exemplary X-ray devices shown in Figs. 1 and 2, may solve one or more of the following problems:
Conventional X-ray imaging results in a purely 2D projective image, which may feature ambiguities that can only be resolved in 3D. Moreover, the quality of the X-ray image may depend strongly on the positioning of the patient relative to the detector and X-ray source. The X-ray device as disclosed herein provides a new imaging modality can be seen as being in-between purely projective imaging (e.g., X-ray) and full 3D modality (e.g., CT). In addition, the proposed X-ray device may relax the requirements on the exact patient positioning for subtle exams (e.g. ankle lateral) since the radiologist can compensate for mis-positionings after the image acquisition.

3D imaging modalities can resolve such ambiguities but are costly, technically very complex and may come with additional patient dose in the case of CT. The X-ray device as disclosed herein may be used to generate pseudo-3D images of a joint in order to detect and classify bone fractures which would otherwise require the acquisition of a CT scan.

Existing stereo-X-Ray / tomosynthesis techniques may involve several detectors and/or sources or are based on moving parts (e.g., C-arm). Some X-ray systems can be promoted to a stereo modality but this may lead to further configuration times and is less favorable in highly efficient workflows. Existing stereo-X-Ray / tomosynthesis with a single tube and detector may involve at least two or more image acquisitions with the tube being differently orientated to the detector. Existing stereo-X-Ray / tomosynthesis techniques also leads to a higher patient radiation dose. The X-ray device as disclosed herein may be used to generate pseudo-3D images of a joint in order to detect and classify bone fractures which would otherwise require the acquisition of a CT scan. Generally, the proposed X-ray device may allow the radiologists to retrospectively vary the viewing direction of a projective X-ray image in order to resolve ambiguities being present in a single 2D projection image. The proposed X-ray device may be integrated into C-arm systems or CT scanners for e.g., reducing the scanning time by simultaneously acquiring multiple viewing directions for one detector-source position.

Besides stereo exam, also different views on the same anatomy are acquired conventionally (e.g. chest PA and lateral) which leads to higher radiation dose. The X-ray device as disclosed herein may allow simultaneously acquiring multiple viewing directions for one detector-source position.

Current CT / C-arm systems only acquire a single view per detector-source position. The X-ray device as disclosed herein may allow simultaneously acquiring multiple viewing directions for one detector-source position.

According to an embodiment of the present invention, the X-ray device further comprises an X-ray optical device configured to redirect the polychromatic X-ray beam such that the polychromatic X-ray beam is angulated in an energy dependent manner.

The X-ray optical device may comprise a wavelength dispersing element, such as an X-ray prism shown in Fig. 1. The X-ray optical device could alternatively be provided as a diffraction grating, an optical device configured to redirect the polychromatic X-ray beam by utilizing Compton scattering, or combinations of one or more of these elements. This configuration will be described in detail hereinafter and in particular with respect to the example shown in Fig. 1.

According to an embodiment of the present invention, the X-ray optical device comprises one or more of: an X-ray prism, an optical device configured to redirect the polychromatic X-ray beam by utilizing Bragg scattering, and an optical device configured to redirect the polychromatic X-ray beam by utilizing Compton scattering.

According to an embodiment of the present invention, the X-ray detector comprises: a dual- or multi-layer sandwich detector or a photo-countering detector.

In other words, a flat spectral detector is then used to acquire an energy-resolved absorption image. This can be realized by a dual- or multi-layer sandwich detector or a photo-counting detector. In the case of a dual-layer detector, one directly obtains a stereo image form the signals of the detector layers. If more than two layers are involved or a photon-counting detector is used, multiple stereo-images with varying relative angulation of the central-beams are acquired in one shot. In other words, more 3D information is imaged in this scenario. Using a photon-counting detector moreover allows the quasi-continuous variation of this angulation angle (in a certain range) by comparing the absorption images of two chosen energy bands.

According to an embodiment of the present invention, the X-ray detector comprises a plurality of tilted detector layers, each detector layer being sensitive to a respective range of energies.

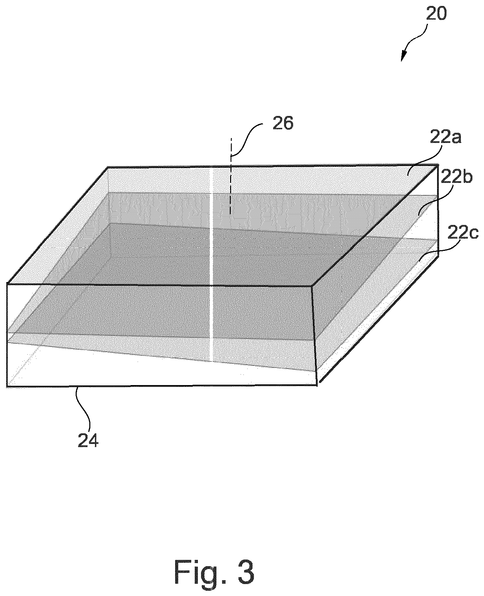

In this configuration, the energy-dependent beam angulation with respect to the X-ray detector is realized by a detector cover hosting two or more tilted detector layers being each sensitive to a certain range of energies. This configuration will be described in detail below and in particular with respect to the examples shown in Figs. 2 and 3.

According to an embodiment of the present invention, the X-ray detector further comprises a mechanical extender in combination with a rotation axis attached to a boundary of one or more detector layers for adjusting a tilt angle of the one or more detector layers.

The mechanical extender in combination with rotation axes attached to the boundary of each layer can be used for symmetrically adjusting the tilt angles of the layers. Thereby, different energy-dependent angulations can be realized. This configuration will be described in detail below and in particular with respect to the examples shown in Fig. 2.

According to a second aspect of the present invention, there is provided an X-ray system. The X-ray system comprises an X-ray device according to the first aspect and any associated example and a computing device. The X-ray device comprises an X-ray generator and an X-ray detector configured acquire a plurality of energy-resolved absorption images of a subject. The computing device is configured to perform image processing of the acquired plurality of energy-resolved absorption images of the subject.

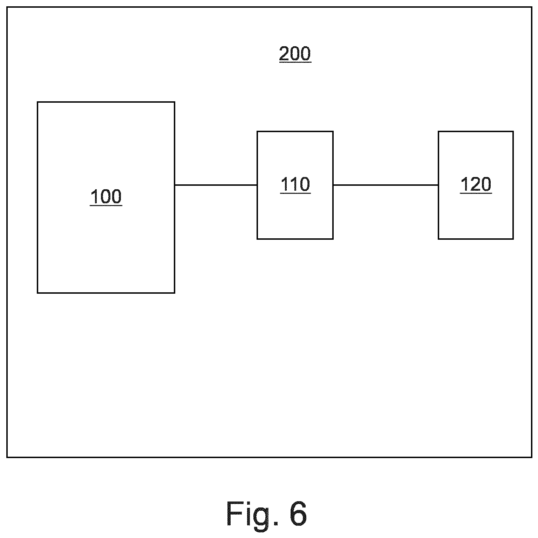

An example of the X-ray system is shown in Fig. 6.

According to an embodiment of the present invention, the computing device is configured to determine viewing directions in different energy bands of the polychromatic X-ray beam, and to associate the viewing directions to respective energy-resolved absorption images.

Accordingly, the detected energy or energy band is converted into a respective viewing direction i.e., angulation of the central beam of X-rays within the given energy or energy band.

According to an embodiment of the present invention, wherein the computing device is configured to fuse two or more of the plurality of energy-resolved absorption images for continuously or quasi-continuously varying the viewing direction.

Accordingly, even if the photon energy is not continuously resolved, the absorption images of the detector layers can be fused within physical approximations e.g., by linear or non-linear combinations for continuously varying the viewing direction in certain limits.

According to an embodiment of the present invention, the computing device is configured to apply a deep-learning model, which has been trained for a given anatomy to estimate a 3D reconstruction from one or more acquired energy-resolved absorption images of the subject.

It is possible to perform 2D-3D reconstruction using 3D statistical shape model (SSM) or generic model. The 2D projections of the 3D SSM or generic model calculated from the training data set may be compared with X-ray images, resulting in a similarity score, and the shape parameters of SSM or generic model may be optimized to maximize the similarity. It is also possible to perform 2D-3D reconstruction using convolutional neural networks (CNNs) to extract anatomical landmarks, and to perform 3D modeling based on the extracted anatomical landmarks.

According to an embodiment of the present invention, the X-ray detector comprises a plurality of tilted detector layers, each detector layer being sensitive to a respective range of energies. The computing device is configured to perform image processing to compensate a distortion caused by a geometrical arrangement of the tilted detector layers.

The geometrical arrangement of the tilted detector layers may lead to distortions. Given a source-detector-cover distance, each detector-layer has effectively a slightly different Source-to-Image Distance (SID), which can be compensated post-hoc (e.g., approximately by an isotropic scaling).

According to an embodiment of the present invention, the computing device is configured to perform image processing to compensate a difference in an energy dependent attenuation behavior of different materials.

For example, it is possible to use a machine-learning algorithm for image-to-image translation which has been trained to compensate the differences in the energy dependent attenuation behaviour. The image-to-image translation may be realized in an unpaired manner using e.g. cycle Generative Adversarial Networks (GANs) or finetuning large models trained in self-supervised manner.

According to an embodiment of the preset invention, the X-ray system further comprises a display configured to display the generated stereo-absorption image of the subject.

According to an embodiment of the present invention, the X-ray system is a C-arm system or a CT system.

According to a third aspect of the present invention, there is provided an X-ray imaging method, comprising:
- generating, by an X-ray generator, a polychromatic X-ray beam in a frequency range for an subject to be imaged, wherein the polychromatic X-ray beam has an energy dependent angulation with respect to the X-ray detector; and
- acquiring, by an X-ray detector, a plurality of energy-resolved absorption images of the subject, which are usable for generating a stereo-absorption image of the subject.

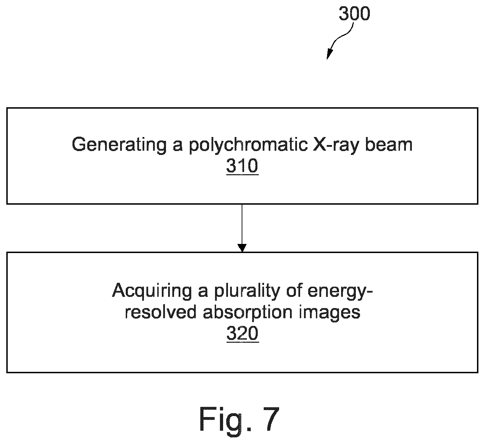

This will be described in detail hereinafter and in particular with respect to the flowchart shown in Fig. 7.

These and other aspects of the present invention will become apparent from and be elucidated with reference to the embodiments described hereinafter.

### BRIEF DESCRIPTION OF THE DRAWINGS

These and other aspects of the invention will be apparent from and elucidated further with reference to the embodiments described by way of examples in the following description and with reference to the accompanying drawings, in which
Fig. 1 illustrates an exemplary X-ray device.
Fig. 2 illustrates a further exemplary X-ray device.
Fig. 3 illustrates a perspective view of an exemplary X-ray detector with three tilted detector layers.
Fig. 4 illustrates a further example of an X-ray device.
Fig. 5A illustrates an exemplary conventional X-ray image of a virtual bone phantom.
Fig. 5B illustrates spectrally generated stereo X-ray images with varying angulations of the central beams of two photon energies.
Fig. 6 illustrates an exemplary X-ray system.
Fig. 7 illustrates a flow chart describing an X-ray imaging method.

It should be noted that the figures are purely diagrammatic and not drawn to scale. In the figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

Fig. 1 illustrates an exemplary X-ray device 100. The X-ray device 100 comprises an X-ray generator 10, an X-ray detector 20, and an X-ray optical device 30.

In the exemplary X-ray device 100 shown in Fig. 1, the X-ray generator 10 is an X-ray tube. The X-ray tube 10 includes a vacuum enclosure 2 having a vacuum interior. The X-ray tube 10 further comprises a cathode 4 including a filament coil and an anode 6 with a target 8. The cathode 4 and the anode 6 are arranged in the vacuum enclosure 2, where the anode 6 faces the filament coil of the cathode 4. An electrical potential difference (e.g., 20 to 150 kV) is applied between the electrodes by a high voltage power supply 12. Electrons are emitted by the cathode 4 and are accelerated by the tube potential through the vacuum to strike the anode 6. On impact with the target 8, electrons that reach the proximity of an atomic nucleus in the target material are decelerated by the positive charge of the nucleus, which result in the conversion of energy into heat (e.g., 99%) and X-ray photons (e.g., 1%). The X-ray photons are released in a polychromatic X-ray beam with a range of energies (also referred to as X-ray spectrum) out of a window (not shown) of the X-ray tube 10 and form the basis for X-ray image formation. The polychromatic X-ray beam may be a cone-shaped beam, a rectangular-shaped beam, a fan-shaped beam, or an X-ray beam in other shapes.

The X-ray optical device 30 is adapted to receive the polychromatic X-ray beam from the X-ray tube 10, and to separate the beam of X-rays into a plurality of energies of X-rays each directed along a respective direction. The X-ray optical device 30 is further operative to direct the plurality of energies of X-rays in a plurality of angular directions and/or displacements with respect to a central beam axis 14 of the polychromatic X-ray beam. Thus, the polychromatic X-ray beam is angulated in an energy dependent manner. In some examples, the X-ray optical device 30 may include a wavelength dispersing element, such as an X-ray prism shown in Fig. 1. The X-ray optical device 30 could alternatively be provided as a diffraction grating, an optical device configured to redirect the polychromatic X-ray beam by utilizing Compton scattering, or combinations of one or more of these elements.

The X-ray optical device 30 directs X-rays at different energies along paths, which are at an angle with respect to the central beam axis 14. Each angle is determined by the X-ray optical device 30. By way of example, as shown in Fig. 1, the X-ray optical device 30 performs spectral separation and generates two X-ray beams including a first X-ray beam 16 at a first energy (e.g., 80 keV) and a second X-ray beam 18 at a second energy (e.g., 140 keV). The first X-ray beam 16 has a central beam 16a and the second X-ray beam 18 has a central beam 18a. As shown in Fig. 1, the angulations of the central beams 16a and 18a relative to the X-ray detector 20 are different, thereby creating two viewing directions for acquiring an X-ray image of the subject. The subject may be a human body or a portion of the human body In other words, the angulation of the central beams represents the difference between the angles at which two plain X-ray images or monocular views are captured. This angle may be applied to construct two views with binocular disparity.

The X-ray detector 20 may be a flat spectral detector used to acquire energy-resolved images. The flat spectral detector may be realized by a dual-or multi-layer sandwich detector or a photon-counting detector. In the case of a dual-layer detector, one directly obtains a stereo image form the signals of the detector layers. If more than two layers are involved or a photon-counting detector is used, multiple stereo-images with varying relative angulation of the central-beams are acquired in one shot. In other words, more 3D information is imaged in this scenario. Using a photon-counting detector may allow the quasi-continuous variation of this angulation angle in a certain range energy comparing the absorption images of two or more chosen energy bands. For example, the photon-counting detector may be configured to classify photons passing through the subject into a number of energy bins in accordance with energy of the photons, and may allow the quasi-continuous variation of this angulation angle in a certain range energy by selecting different energy bins.

Often the imaging apparatus is configured such that the central beam hits the detector at an angle of 90°. In the illustrated example shown in Fig. 1, there is no single central beam anymore. In one example, the X-ray device 100 may be configured to deviate symmetrically from the central beam being normal to the detector plane so that the X-ray image can be viewed symmetrically from "both sides" with respect to the detector normal. For example, the X-ray generator 10 may be configured to generate the polychromatic X-ray beam with the central beam axis 14 having a small tilt angle with respect to the normal of the detector plane of the X-ray detector 20.

Fig. 2 illustrates a further exemplary X-ray device 100. In the example shown in Fig. 2, the X-ray device 100 comprises an X-ray generator 10 and an X-ray detector 20. The X-ray generator 10 shown in Fig. 2 and the X-ray generator 10 shown in Fig. 1 may have similar components. Unlike the exemplary X-ray device shown in Fig. 1, the X-ray device 100 shown in Fig. 2 does not comprise an X-ray optical device adapted to separate the beam of X-rays into a plurality of energies of X-rays. Instead, in the exemplary X-ray device 100 shown in Fig. 2, the energy-dependent beam angulation with respect to the X-ray detector 20 is realized by a detector cover hosting two or more tilted detector layers, such as first tilted detector layer 22a, and second tilted detector layer 22b shown in Fig. 2. The two or more tilted detector layers are each sensitive to a certain range of energies. For example, the first tilted detector layer 22a may be sensitive to a range of energies around 80 keV, and the second tilted detector layer 22b may be sensitive to a range of energies around 100 keV.

Although Fig. 2 may show a titled dual-layer detector by way of example, it will be appreciated that the X-ray detector 20 may comprise three or more detector layers being each sensitive to a certain range of energies. For example, Fig. 3 shows a perspective view of an exemplary X-ray detector 20 with three detector layers including a first detector layer 20a, a second detector layer 20b, and a third detector layer 20c. The first detector layer 20a, the second detector layer 20b, and the third detector layer 20c are each sensitive to a certain range of energies. For example, the first detector layer 20a is sensitive to X-rays with an energy of 80 keV, the second detector layer 20b is sensitive to X-rays with an energy of 100 keV, and the third detector layer 20c is sensitive to X-ray with an energy of 140 keV. In the exemplary X-ray detector 20 shown in Fig. 3, the first detector layer 20a is parallel to a detector cover 24 of the X-ray detector 20 and therefore orthogonal to an orthogonal axis 26 of the X-ray detector 20. The second detector layer 20b and the third detector layer 20c are tilted with respect to the orthogonal axis 26 of the X-ray detector 20 and with respect to each other so that no pair of detector layers 20a, 20b and 20c are parallel. In other words, the detector layer 20a is neither parallel to the detector layer 20b nor to the detector layer 20c, and the detector layer 20b is not parallel to the detector layer 20c). This exemplary X-ray detector 20 may allow to generate stereo images with retrospectively tunable viewing directions. It is noted that the exemplary X-ray detector 20 shown in Fig. 3 is provided as an example. It will be appreciated that the X-ray detector 20 may comprise four or more detector layers, and each layer may be tilted with respect to the detector cover 24 and/or the orthogonal axis 26 at any suitable angle.

Turning back to Fig. 2, in some implementations, a mechanical extender 28 may be provided in combination with rotation axes 30 attached to the boundary of one or more detector layers for symmetrically or alternatively asymmetrically adjusting the tilt angles of one or more of the detector layers, such as the first and second detector layers 22a and 22b shown in Fig. 2. Thereby, different energy-dependent angulations can be realized.

With the X-ray device, such as the exemplary X-ray devices 100 shown in Figs. 1 and 2, a plurality of energy-resolved absorption images with different angulations may be acquired. The acquired energy-resolved absorption images with two different angulations may be used as left-eye and right-eye views and may be horizontally merged into single left-right 3D images. Stereo vision may be achieved using Virtual Reality (VR) glasses, e.g., a simple VR box, to observe the merged images. Alternatively, the acquired energy-resolved absorption images with two different angulations may be translated into single red-blue 3D images, and red-blue glasses may be used to observe the edited images, generating stereo vision. As a further option, the acquired energy-resolved absorption images with two different angulations may be translated into interlaced X-ray stereoscopic images and displayed with a naked-eye 3D device.

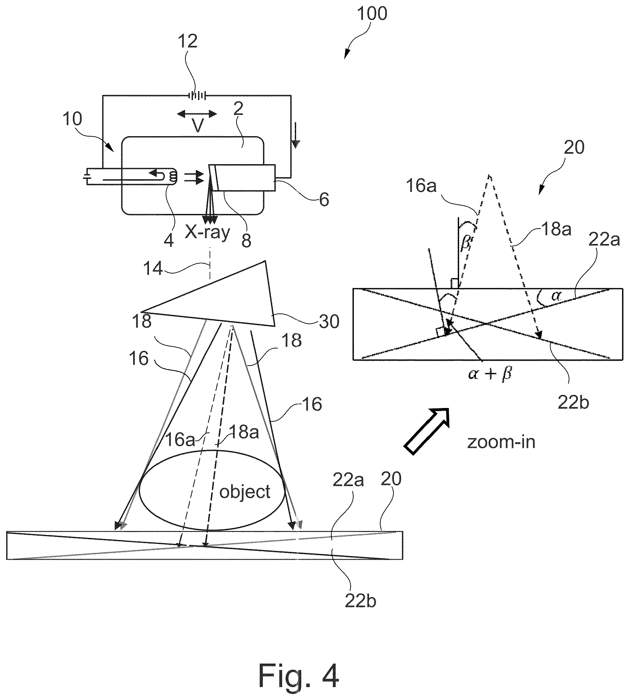

Fig. 4 illustrates a further example of an X-ray imaging device 100. In the example shown in Fig. 4, the X-ray device 100 comprises an X-ray generator 10 and an X-ray detector 20. The X-ray generator 10 shown in Fig. 4 and the X-ray generator 10 shown in Figs. 1 and 2 may have similar components.

Unlike the exemplary X-ray device shown in Figs. 1 and 2, the X-ray device 100 shown in Fig. 4 comprises the X-ray optical device 30 shown in Fig. 1 adapted to separate the beam of X-rays into a plurality of energies of X-rays and the X-ray detector 20 shown in Fig. 2 hosting two or more tilted detector layers, such as the first and second detector layers 22a and 22b shown in Fig. 4. In other words, in this illustrated example, the energy-dependent beam angulation with respect to the X-ray detector 20 is achieved by both the X-ray optical device 30 and the tilted detector layers (e.g., first and second detector layers 22a and 22b shown in Fig. 4). Each detector layer being sensitive to a certain energy band is tilted in such a way that the resulting viewing directions of energy-dependent absorption images cover a larger range as compared to the embodiment corresponding to Fig. 1, which does not utilize tilted detector layers. For example, as shown in Fig. 4, the first X-ray beam 16 at the first energy enters the X-ray detector 20 at an incident angle *β*. The incident angle *β* is an angle between the central beam 16a of the first X-ray beam 16 and the line perpendicular (at 90 degree angle) to the detector surface at the point of incidence. The detector layer 22a is sensitive to the first energy and is tilted at an angle of α (where α≠0°) with respect to the detector surface. The effective viewing direction of the first X-ray beam 16 is measured by the angle between its central beam 16a and normal of the detector layer being sensitive to this energy, i.e., detector layer 22a. As shown in FIG. 4, the interactions of the X-ray optical device 30 and the tilted detector layer 22a thus create an effective viewing direction at an angle of α+*β* for acquiring an X-ray image of the subject. The detector layer 22b may be used to adjust the effective viewing angle of the second X-ray beam 18 in a similar manner. To summarize, given the energy-dependent redirection of the different energy-bands by the X-ray optical device 30, the first and second tilted detector layers 22a and 22b may be configured so that the minimal and the maximal (signed) angle of incidence are smaller and larger as compared to the minimal and maximal angle of incidence in the case for the embodiment corresponding to Fig. 1, respectively. In this way, it is possible to simultaneously acquire multiple viewing directions within a larger range for one detector-source position compared to the embodiments shown in Figs. 1 and 2.

Although not shown in Fig. 4, it will be appreciated that the mechanical extender 28 shown in Fig. 2 may also be used in the embodiment of Fig. 4 for symmetrically or alternatively asymmetrically adjusting the tilt angles of one or more of the detector layers, such as the first and second detector layers 22a and 22b shown in Fig. 2. Thereby, different energy-dependent angulations can be realized.

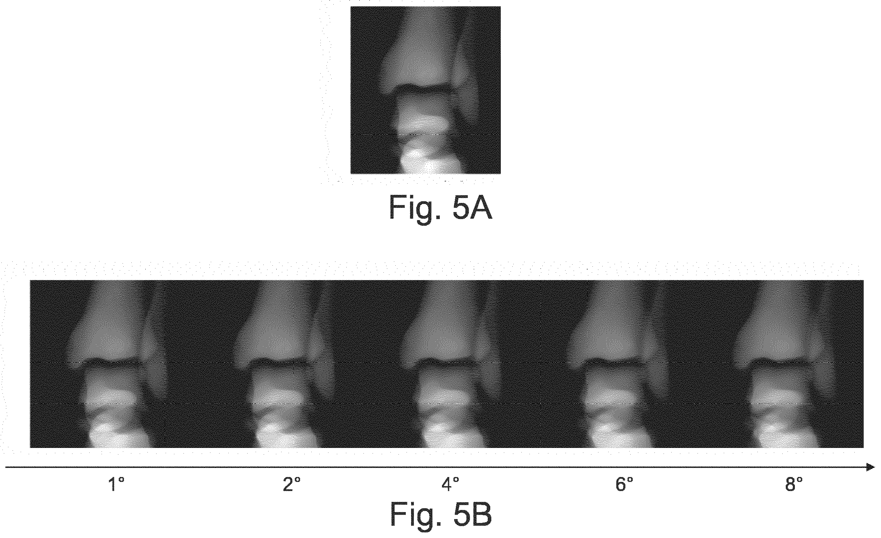

Fig. 5A illustrates an exemplary conventional X-ray image of a virtual bone phantom. Fig. 5B illustrates spectrally generated stereo X-ray images with varying angulations of the central beams of two photon energies. The angulation is represented as the angle (e.g., 1°, 2°, 4°, 6°, and 8° shown in Fig. 5B) with respect to the central beam axis 14 shown in Figs. 1 and 2. Each stereo X-ray image in Fig. 5B is a single red-blue 3D image and red-blue glasses may be best seen in color used to observe these stereo X-ray images . Although the stereo X-ray images in Fig. 5B are presented in black and white, it will be appreciated that these spectrally generated stereo X-ray images may also be presented as colored images. For example, in Fig. 5B the spectrally generated stereo X-ray images with varying angulations of the central beams of two photon energies may be represented by red-green color encoding. The color encoding may be used to discern X-ray images acquired at different angulations (i.e., different energies).

The X-ray device as disclosed herein may have one or more of the following potential clinical applications:
This new imaging modality may be seen as being in-between purely projective imaging (e.g., X-ray) and full 3D modality (e.g., CT). Thereby, it may be used in an early stage of the diagnostic pathway for clarifying indications without the need for acquiring full 3D images. For example, it may be used to generate pseudo-3D images of a joint in order to detect and classify bone fractures which would otherwise require the acquisition of a CT scan with higher dose, costs of operation, etc. Generally, the proposed X-ray device may allow the radiologists to retrospectively vary the viewing direction of a projective X-ray image in order to resolve ambiguities being present in a single 2D projection image.

The proposed X-ray device may relax the requirements on the exact patient positioning for subtle exams (e.g., ankle lateral) since the radiologist can compensate for mis-positionings after the image acquisition.

Using the proposed X-ray device in mobile X-ray systems may be particularly useful, as positioning is much more difficult here (e.g., for bedside patients) so that a retrospective correction of the viewing direction can by highly valuable.

Besides facilitating a pseudo-3D view, also the spectral information may be used for material composition for diagnostic purposes. For example, energy resolution can enable to distinguish particular properties of soft tissues (e.g., differentiation of odema from pathological tissue swelling), which are currently only distinguishable via indirect inference during radiological interpretation.

The proposed X-ray device may also be integrated into C-arm systems or CT scanners for e.g. reducing the scanning time by simultaneously acquiring multiple viewing directions for one detector-source position.

Fig. 6 illustrates an exemplary X-ray system 200. Examples of the X-ray system 200 may include, but are not limited to, C-arm systems and CT systems. The exemplary X-ray system 200 comprises an X-ray device 100 and a computing device 110. The exemplary X-ray system may optionally comprise a display 120.

The X-ray device 100 comprises an X-ray generator and an X-ray detector configured acquire a plurality of energy-resolved absorption images of a subject. Two exemplary X-ray devices 100 are shown in Fig. 1 and Fig. 2.

The computing device 110 may be implemented in numerous ways (e.g., such as with dedicated hardware) to perform the functions described herein. A "processor" is one example of the computing device 110, which employs one or more microprocessors that may be programmed using software (e.g., microcode) to perform various functions described herein. The computing device 110 may be implemented with or without employing a processor, and also may be implemented as a combination of dedicated hardware to perform some functions and a processor (e.g., one or more programmed microprocessors and associated circuitry) to perform other functions. Examples of computing device components that may be employed in various embodiments of the present disclosure include, but are not limited to, conventional microprocessors, application specific integrated circuits (ASICs), and field-programmable gate arrays (FPGAs). In various implementations, a processor may be associated with one or more storage media (generically referred to herein as "memory," e.g., volatile and non-volatile computer memory). In some implementations, the storage media may be encoded with one or more programs that, when executed on one or more processors and/or controllers, perform at least some of the functions described herein. Various storage media may be fixed within the computing device or may be transportable, such that the one or more programs stored thereon can be loaded into the computing device so as to implement various aspects of the present disclosure described herein. The terms "program" or "computer program" are used herein in a generic sense to refer to any type of computer code (e.g., software or microcode) that can be employed to program one or more processors.

The computing device 110 is configured to receive the plurality of acquired energy-resolved absorption images of the subject e.g., from the X-ray detector 20 shown in Figs. 1 and 2, and perform image processing of the acquired plurality of energy-resolved absorption images of the subject for generating a stereo-absorption image of the subject. For example, the computing device 110 may perform one or more processing steps including, but not limited to, compensation for perspective distortion, compensation of energy dependent attenuation behavior of different materials, denoising the energy-resolved absorption images (in particular for photo-counting detectors), association of energy-resolved absorption images with viewing directions, identification of material composition, and rendering of stereo-images. These exemplary processing steps will be described in detail below.

In some examples, the computing device 110 may perform image processing to compensate perspective distortion. For example, in the exemplary X-ray device 100 shown in Fig. 2, the X-ray detector 20 comprises a plurality of tilted detector layers, each detector layer being sensitive to a respective range of energies. The geometrical arrangement of the detector layers of such X-ray detector 20 may lead to distortions. In such cases, the computing device 110 may be configured to perform image processing to compensate a distortion caused by a geometrical arrangement of the tilted detector layers. For example, given a source-detector-cover distance, each detector layer has effectively a slightly different SID, which can be compensated post-hoc e.g., approximately by an isotropic scaling.

In some examples, the computing device 110 may perform imaging processing to compensate a difference in an energy dependent attenuation behavior of different materials, e.g., the attenuation via the photo-electric scales like (Z/E)³ with the atomic number Z and the energy E. For example, a machine-learning algorithm may be used for image-to-image translation, which has been trained to compensate the differences in the energy dependent attenuation behavior. This is feasible due to the only slight variation of the viewing direction in different energy bands and the thereby obtained redundant image information. The image-to-image translation may be realized in an unpaired manner using e.g. cycle GANs or fine-tuning large models trained in self-supervised manner. If the X-ray detector is a photon-counting detector used in the X-ray device 100 shown in Fig. 1, it is possible to quasi continuously select small energy bands and correspondingly vary the viewing direction. This may allow compensating the energy-dependent attenuation behavior iteratively by sliding in small bins over the measured energy range.

As the number of photons in each energy bin will be much less as compared to a conventional X-ray image for bounding the radiation dose for the patient, the signal-to-noise ratio of the energy-resolved absorption images may be lower as compared to conventional X-ray images. In some examples, the computing device 110 may perform imaging processing to denoise the energy-resolved absorption images. This can be realized by e.g. classical denoising algorithms taking the physics of the noise into account or by a machine learning model, e.g. a deep neural network, trained to denoise X-ray images in supervised manner.

In some examples, the computing device 110 may be configured to determine viewing directions in different energy bands of the polychromatic X-ray beam, and to associate the viewing directions to respective energy-resolved absorption images. Depending on the details of the realizations, such as the realizations shown in Figs.1 and 2, the detected energies or energy bands are converted into respective viewing directions, i.e., angulation of the central beam of X-rays within a given energy or energy band. This conversion may be based on a mathematical-physical model, taking the source and detector configurations, and in the case of the realization option shown in Fig. 1, the physical properties of the employed X-rays optical device into account.

In some examples, the computing device 110 may be configured to perform rendering of stereo-images. For example, as described hereinbefore, a plurality of energy-resolved absorption images may be acquired with different angulation. The resulting images at these angulations may be used as left-eye and right-eye views and may be horizontally merged into single left-right 3D images displayed on a display 120. VR glasses may be used for achieving stereo vision. Observation with VR glasses can produce realistic stereo views of an anatomical structure of the subject. In some examples, the display 120 may be a naked-eye device, and the resulting images at these angulations may be translated into interlaced X-ray stereoscopic images by the computing device 110 and displayed with the naked-eye 3D device.

In some examples, a radiologist may retrospectively tune the viewing direction in order to resolve ambiguities being present in a single 2D projective image or in order to correct for suboptimal patient positioning. For example, if the X-ray detector is a dual- or multi-layer sandwich detector, the photon energy is not continuously resolved. In such cases, the absorption images of the detector layers of the X-ray detector may be fused by the computing device 110 within a physical approximations, e.g., by a linear or non-linear combination, for continuously or quasi-continuously varying the viewing direction. If the X-ray detector has a doubly angulated detector layer configuration, such as the X-ray detector shown in Fig. 3, it is possible to change the viewing direction by rotating about the two axes of the detector within certain limits. By combining the absorption images of the three energy bands, the viewing direction can be continuously adjusted post-hoc. If the X-ray detector is a photon-counting detector configured to classify photons passing through the subject into a number of energy bins in accordance with energy of the photons, the computing device 110 may be configured to continuously or quasi-continuously vary the viewing direction by selecting different energy bins.

In some examples, the computing device 110 may apply a deep-learning model, which has been trained for a given anatomy to estimate a 3D reconstruction from one or more acquired energy-resolved absorption images of the subject. In other words, a 3D shape of the anatomy may be estimated and constructed from one or more 2D energy-resolved absorption images. For example, in exemplary methods for estimating the 3D bone shape underlying X-ray images, the 2D projections of the 3D statistical shape model (SSM) or generic model calculated from the training data set may be compared with X-ray images, resulting in a similarity score, and the shape parameters of SSM or generic model may be optimized to maximize the similarity. It is also possible to perform 2D-3D reconstruction using CNNs to extract anatomical landmarks, and to perform 3D modeling based on the extracted anatomical landmarks.

In some examples, the computing device 110 may use spectral information for material composition in slightly angulated views. By an algorithmic compensation of the different views corresponding to different energies, e.g., via a machine-learning model trained for a certain anatomy, also a conventional material composition for a single view may be supported.

Fig. 7 illustrates a flowchart describing an X-ray imaging method 300.

At block 310, an X-ray generator generates a polychromatic X-ray beam in a frequency range for a subject to be imaged. The polychromatic X-ray beam has an energy dependent angulation with respect to the X-ray detector. The energy-dependent angulation may be achieved by the exemplary configurations shown in Figs. 1 and 2.

At bock 320, an X-ray detector acquires a plurality of energy-resolved absorption images of the subject, which are usable for generating a stereo-absorption image of the subject.

In the exemplary X-ray device shown in Fig. 1, the X-ray detector may be a flat spectral detector used to acquire the energy-resolved absorption images. A flat spectral detector is then used to acquire an energy-resolved absorption image. The flat spectral detector may be realized by a dual- or multi-layer sandwich detector or a photo-counting detector. In the case of a dual-layer detector, one directly obtains a stereo image form the signals of the detector layers. If more than two layers are involved or a photon-counting detector is used, multiple stereo-images with varying relative angulation of the central-beams are acquired in one shot. In other words, more 3D information is imaged in this scenario. Using a photon-counting detector moreover allows the quasi-continuous variation of this angulation angle (in a certain range) by comparing the absorption images of two chosen energy bands.

In the exemplary X-ray device shown in Fig. 2, the X-ray detector may comprise two or more tilted detector layers, being each sensitive to a certain range of energies. An exemplary X-ray detector with two tilted detector layers is shown in Fig. 2, and an exemplary X-ray detector layer with three tilted detector layers is shown in Fig. 3.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this patent. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An X-ray device (100), comprising:
- an X-ray generator (10); and
- an X-ray detector (20);
wherein the X-ray generator is configured to generate a polychromatic X-ray beam in a frequency range for an subject to be imaged;
wherein the polychromatic X-ray beam is configured to have an energy dependent angulation with respect to the X-ray detector; and
wherein the X-ray detector is configured to acquire a plurality of energy-resolved absorption images of the subject, which are usable for generating a stereo-absorption image of the subject.

2. The X-ray device according to claim 1, further comprising:
- an X-ray optical device (30) configured to redirect the polychromatic X-ray beam such that the polychromatic X-ray beam is angulated in an energy dependent manner.

3. The X-ray device according to claim 2, wherein the X-ray optical device comprises one or more of:
- an X-ray prism;
- an optical device configured to redirect the polychromatic X-ray beam by utilizing Bragg scattering; and
- an optical device configured to redirect the polychromatic X-ray beam by utilizing Compton scattering.

4. The X-ray device according to claim 2 or 3, wherein the X-ray detector comprises:
- a dual- or multi-layer sandwich detector; or
- a photo-countering detector.

5. The X-ray device according to any one of claims 1 to 3, wherein the X-ray detector comprises a plurality of tilted detector layers (22a, 22b, 22c), each detector layer being sensitive to a respective range of energies.

6. The X-ray device according to claim 5,
wherein the X-ray detector further comprises a mechanical extender (28) in combination with a rotation axis (30) attached to a boundary of one or more detector layers for adjusting a tilt angle of the one or more detector layers.

7. An X-ray system (200), comprising:
- an X-ray device (100) according to any one of the preceding claims; and
- a computing device (110);
wherein the X-ray device comprises an X-ray generator and an X-ray detector configured acquire a plurality of energy-resolved absorption images of a subject; and
wherein the computing device is configured to perform image processing of the plurality of acquired energy-resolved absorption images of the subject.

8. The X-ray system according to claim 7,
wherein the computing device is configured to determine viewing directions in different energy bands of the polychromatic X-ray beam, and to associate the viewing directions to respective energy-resolved absorption images.

9. The X-ray system according to claim 8,
wherein the computing device is configured to fuse two or more of the plurality of energy-resolved absorption images for continuously or quasi-continuously varying the viewing direction.

10. The X-ray system according to any one of claims 7 to 9,
wherein the computing device is configured to apply a deep-learning model, which has been trained for a given anatomy to estimate a three-dimensional, 3D, reconstruction from one or more acquired energy-resolved absorption images of the subject.

11. The X-ray system according to any one of claims 7 to 10,
wherein the X-ray detector comprises a plurality of tilted detector layers, each detector layer being sensitive to a respective range of energies; and
where the computing device is configured to perform image processing to compensate a distortion caused by a geometrical arrangement of the tilted detector layers.

12. The X-ray system according to any one of claims 7 to 11,
wherein the computing device is configured to perform image processing to compensate a difference in an energy dependent attenuation behavior of different materials.

13. The X-ray system according to any one of claims 7 to 12, further comprising:
- a display (120) configured to display the generated stereo-absorption image of the subject.

14. The X-ray system according to any one of claims 7 to 13,
wherein the X-ray system is a C-arm system or a Computed Tomography (CT) system.

15. An X-ray imaging method (300), comprising:
- generating (310), by an X-ray generator, a polychromatic X-ray beam in a frequency range for an subject to be imaged, wherein the polychromatic X-ray beam has an energy dependent angulation with respect to the X-ray detector; and
- acquiring (320), by an X-ray detector, a plurality of energy-resolved absorption images of the subject, which are usable for generating a stereo-absorption image of the subject.

## Patentansprüche

1. Röntgenvorrichtung (100), umfassend:
- einen Röntgengenerator (10); und
- einen Röntgendetektor (20);
wobei der Röntgengenerator konfiguriert ist, um einen polychromatischen Röntgenstrahl in einem Frequenzbereich für eine abzubildende Zielperson zu generieren;
wobei der polychromatische Röntgenstrahl konfiguriert ist, um in Bezug auf den Röntgendetektor eine energieabhängige Angulation aufzuweisen; und
wobei der Röntgendetektor konfiguriert ist, um eine Vielzahl von energieaufgelösten Absorptionsbildern der Zielperson zu erfassen, die zum Generieren eines Stereo-Absorptionsbildes der Zielperson verwendbar sind.

2. Röntgenvorrichtung nach Anspruch 1, weiter umfassend:
- eine optische Röntgenvorrichtung (30), die konfiguriert ist, um den polychromatischen Röntgenstrahl derart umzulenken, dass der polychromatische Röntgenstrahl auf eine energieabhängige Weise anguliert wird.

3. Röntgenvorrichtung nach Anspruch 2, wobei die optische Röntgenvorrichtung eines oder mehrere umfasst von:
- einem Röntgenprisma;
- einer optischen Vorrichtung, die konfiguriert ist, um den polychromatischen Röntgenstrahl durch Nutzung von Bragg-Streuung umzulenken; und
- eine optische Vorrichtung, die konfiguriert ist, um den polychromatischen Röntgenstrahl durch Nutzen von Compton-Streuung umzulenken.

4. Röntgenvorrichtung nach Anspruch 2 oder 3, wobei der Röntgendetektor umfasst:
- einen zwei- oder mehrschichtigen Sandwich-Detektor; oder
- einen photonenzählenden Detektor.

5. Röntgenvorrichtung nach einem der Ansprüche 1 bis 3, wobei der Röntgendetektor eine Vielzahl von geneigten Detektorschichten (22a, 22b, 22c) umfasst, wobei jede Detektorschicht für einen jeweiligen Energiebereich empfindlich ist.

6. Röntgenvorrichtung nach Anspruch 5,
wobei der Röntgendetektor weiter ein mechanisches Verlängerungselement (28) in Kombination mit einer Drehachse (30) umfasst, die zum Anpassen eines Neigungswinkels der einen oder mehreren Detektorschichten an einer Grenzfläche der einen oder mehreren Detektorschichten angegliedert ist.

7. Röntgensystem (200), umfassend:
- eine Röntgenvorrichtung (100) nach einem der vorstehenden Ansprüche; und
- eine Rechenvorrichtung (110);
wobei die Röntgenvorrichtung einen Röntgengenerator und einen Röntgendetektor umfasst, die konfiguriert sind, um eine Vielzahl von energieaufgelösten Absorptionsbildern einer Zielperson zu erfassen; und
wobei die Rechenvorrichtung konfiguriert ist, um eine Bildverarbeitung der Vielzahl von erfassten energieaufgelösten Absorptionsbildern der Zielperson durchzuführen.

8. Röntgensystem nach Anspruch 7,
wobei die Rechenvorrichtung konfiguriert ist, um die Blickrichtungen in unterschiedlichen Energiebändern des polychromatischen Röntgenstrahls zu bestimmen und die Blickrichtungen mit den jeweiligen energieaufgelösten Absorptionsbildern zu assoziieren.

9. Röntgensystem nach Anspruch 8,
wobei die Rechenvorrichtung konfiguriert ist, um zwei oder mehr der Vielzahl von energieaufgelösten Absorptionsbildern zum kontinuierlichen oder quasi-kontinuierlichen Variieren der Blickrichtung zu fusionieren.

10. Röntgensystem nach einem der Ansprüche 7 bis 9,
wobei die Rechenvorrichtung konfiguriert ist, um ein Deep-Learning-Modell anzuwenden, das für eine gegebene Anatomie trainiert wurde, um eine dreidimensionale, 3D-, Rekonstruktion aus einem oder mehreren erfassten energieaufgelösten Absorptionsbildern der Zielperson abzuschätzen.

11. Röntgensystem nach einem der Ansprüche 7 bis 10,
wobei der Röntgendetektor eine Vielzahl von geneigten Detektorschichten umfasst, wobei jede Detektorschicht für einen jeweiligen Energiebereich empfindlich ist; und
wobei die Rechenvorrichtung konfiguriert ist, um eine Bildverarbeitung durchzuführen, um eine Verzerrung auszugleichen, die durch eine geometrische Anordnung der geneigten Detektorschichten verursacht wird.

12. Röntgensystem nach einem der Ansprüche 7 bis 11,
wobei die Rechenvorrichtung konfiguriert ist, um eine Bildverarbeitung durchzuführen, um einen Unterschied in einem energieabhängigen Abschwächungsverhalten unterschiedlicher Materialien auszugleichen.

13. Röntgensystem nach einem der Ansprüche 7 bis 12, weiter umfassend:
- eine Anzeige (120), die konfiguriert ist, um das generierte Stereo-Absorptionsbild der Zielperson anzuzeigen.

14. Röntgensystem nach einem der Ansprüche 7 bis 13,
wobei das Röntgensystem ein C-Bogen-System oder ein Computertomographie-(CT-) System ist.

15. Röntgenbildgebungsverfahren (300), umfassend:
- Generieren (310) eines polychromatischen Röntgenstrahls in einem Frequenzbereich für eine abzubildende Zielperson durch einen Röntgengenerator, wobei der polychromatische Röntgenstrahl eine energieabhängige Angulation in Bezug auf den Röntgendetektor aufweist; und
- Erfassen (320) einer Vielzahl von energieaufgelösten Absorptionsbildern der Zielperson durch einen Röntgendetektor, die zum Generieren eines Stereo-Absorptionsbildes der Zielperson verwendbar sind.

## Revendications

1. Dispositif à rayons X (100), comprenant :
- un générateur de rayons X (10) ; et
- un détecteur de rayons X (20) ;
dans lequel le générateur de rayons X est configuré pour générer un faisceau de rayons X polychromatique dans une plage de fréquences pour un sujet à imager ;
dans lequel le faisceau de rayons X polychromatique est configuré pour présenter une angulation dépendant de l'énergie par rapport au détecteur de rayons X ; et
dans lequel le détecteur de rayons X est configuré pour acquérir une pluralité d'images d'absorption à résolution d'énergie du sujet, qui sont utilisables pour générer une image d'absorption stéréo du sujet.

2. Dispositif à rayons X selon la revendication 1, comprenant en outre :
- un dispositif optique à rayons X (30) configuré pour rediriger le faisceau de rayons X polychromatique de sorte que le faisceau de rayons X polychromatique soit angulé d'une manière dépendante de l'énergie.

3. Dispositif à rayons X selon la revendication 2, dans lequel le dispositif optique à rayons X comprend un ou plusieurs de :
- un prisme à rayons X ;
- un dispositif optique configuré pour rediriger le faisceau de rayons X polychromatique en utilisant la diffusion de Bragg ; et
- un dispositif optique configuré pour rediriger le faisceau de rayons X polychromatique en utilisant la diffusion Compton.

4. Dispositif à rayons X selon la revendication 2 ou 3, dans lequel le détecteur de rayons X comprend :
- un détecteur sandwich à double ou multicouche ; ou
- un détecteur à comptage de photons.

5. Dispositif à rayons X selon l'une quelconque des revendications 1 à 3, dans lequel le détecteur de rayons X comprend une pluralité de couches de détecteur inclinées (22a, 22b, 22c), chaque couche de détecteur étant sensible à une plage respective d'énergies.

6. Dispositif à rayons X selon la revendication 5,
dans lequel le détecteur de rayons X comprend en outre un extenseur mécanique (28) en combinaison avec un axe de rotation (30) fixé à une limite d'une ou plusieurs couches de détecteur pour ajuster un angle d'inclinaison de la ou des couches de détecteur.

7. Système à rayons X (200), comprenant :
- un dispositif à rayons X (100) selon l'une quelconque des revendications précédentes ; et
- un dispositif informatique (110) ;
dans lequel le dispositif à rayons X comprend un générateur de rayons X et un détecteur de rayons X configurés pour acquérir une pluralité d'images d'absorption à résolution d'énergie d'un sujet ; et
dans lequel le dispositif informatique est configuré pour effectuer le traitement d'images de la pluralité d'images d'absorption à résolution d'énergie acquises du sujet.

8. Système à rayons X selon la revendication 7,
dans lequel le dispositif informatique est configuré pour déterminer les directions de vision dans différentes bandes d'énergie du faisceau de rayons X polychromatique, et pour associer les directions de vision aux images d'absorption à résolution d'énergie respectives.

9. Système à rayons X selon la revendication 8,
dans lequel le dispositif informatique est configuré pour fusionner deux ou plusieurs des images d'absorption à résolution d'énergie pour faire varier de manière continue ou quasi continue la direction de visualisation.

10. Système à rayons X selon l'une quelconque des revendications 7 à 9,
dans lequel le dispositif informatique est configuré pour appliquer un modèle d'apprentissage profond, qui a été entraîné pour une anatomie donnée, afin d'estimer une reconstruction tridimensionnelle (3D) à partir d'une ou plusieurs images d'absorption à résolution d'énergie acquises du sujet.

11. Système à rayons X selon l'une quelconque des revendications 7 à 10,
dans lequel le détecteur de rayons X comprend une pluralité de couches de détecteur inclinées, chaque couche de détecteur étant sensible à une plage d'énergies respective ; et
où le dispositif informatique est configuré pour effectuer un traitement d'image afin de compenser une distorsion causée par un agencement géométrique des couches de détecteurs inclinées.

12. Système à rayons X selon l'une quelconque des revendications 7 à 11,
dans lequel le dispositif informatique est configuré pour effectuer un traitement d'image afin de compenser une différence dans un comportement d'atténuation dépendant de l'énergie de différents matériaux.

13. Système à rayons X selon l'une quelconque des revendications 7 à 12, comprenant en outre :
- un écran (120) configuré pour afficher l'image d'absorption stéréo générée du sujet.

14. Système à rayons X selon l'une quelconque des revendications 7 à 13,
dans lequel le système à rayons X est un système à bras en C ou un système de tomodensitométrie (TDM).

15. Procédé d'imagerie par rayons X (300), comprenant :
- la génération (310), par un générateur de rayons X, d'un faisceau de rayons X polychromatique dans une plage de fréquences pour un sujet à imager, dans lequel le faisceau de rayons X polychromatique présente une angulation dépendant de l'énergie par rapport au détecteur de rayons X ; et
- l'acquisition (320), par un détecteur de rayons X, d'une pluralité d'images d'absorption à résolution d'énergie du sujet, qui sont utilisables pour générer une image d'absorption stéréo du sujet.
